# EUROPEAN PATENT SPECIFICATION

(11) **EP 3 712 226 B1**
(45) Date of publication and mention of the grant of the patent: **24.05.2023**
(21) Application number: 17932005.6
(22) Date of filing: 16.11.2017
(51) Int. Cl.: C09K 3/30, A61K 8/06

(54) **CRACKING AEROSOL COMPOSITION**
CRACKING-AEROSOLZUSAMMENSETZUNG
COMPOSITION D'AÉROSOL DE CRAQUAGE

(43) Date of publication of application: 23.09.2020
(73) Proprietor: Toyo Aerosol Industry Co., Ltd., Tokyo 141-0022 (JP)
(72) Inventor: KAMIJYO, Hokuto, Tokyo 141-0022 (JP); ISHIDA, Shohei, Tokyo 141-0022 (JP)
(74) Representative: dompatent von Kreisler Selting Werner - Partnerschaft von Patent- und Rechtsanwälten mbB
(86) International application number: PCT/JP2017/041239
(87) International publication number: WO 2019/097622

(56) References cited:
- WO-A1-2011/155630
- WO-A1-2012/033196
- JP-A- 2000 345 146
- JP-A- 2009 256 367
- JP-A- 2013 203 663
- JP-A- 2017 100 996
- JP-A- 2017 109 935

## Description

### TECHNICAL FIELD

The present invention relates to a cracking aerosol composition, and more particularly to a cracking aerosol composition which generates a foam-breaking sound with the spontaneous breakage of foam formed by foaming gradually generated in a discharge.

### BACKGROUND ART

As one type of aerosol composition, there is conventionally known a cracking aerosol composition which generates a foam-breaking sound with the spontaneous breakage of foam formed by foaming gradually generated in a discharge (for example, see JP 2017109935, JP 201710099, JP 2009256367, Patent Literature 1 and Patent Literature 2).

Patent Literature 1 and Patent Literature 2 each disclose a cracking aerosol composition including a liquefied petroleum gas, water, a surfactant, an oily component, a lower alcohol and the like, in which two types of surfactants having different HLB values are simultaneously used as the surfactant. It is described that, in these cracking aerosol compositions according to Patent Literature 1 and Patent Literature 2, the content ratio of a propellant can be reduced to not more than 50 mass %. Further, it is described that, in the cracking aerosol composition according to Patent Literature 1, a liquefied petroleum gas may preferably be used alone as the propellant, however, a mixed gas of a liquefied petroleum gas and a compressed gas (for example, carbon dioxide gas) can also be used.

However, when the inventors of the present invention tried to use a liquefied petroleum gas and carbon dioxide gas simultaneously in the cracking aerosol composition of Patent Literature 1, it was revealed that the liquefied petroleum gas was required to be contained in a proportion of not lower than 60 mass % per 100 mass % of the composition in order to obtain sufficient cracking characteristics. Further, when the proportion of the liquefied petroleum gas is increased to not lower than 60 mass % in the cracking aerosol composition, the amount of a liquid concentrate is inevitably relatively reduced, and so the number of usable times of an aerosol product prepared by filling such a cracking aerosol composition into an aerosol container tends to decrease. WO 2012/033196 and WO 2011/155630 disclose aerosol compositions.

### Citation List

### Patent Literature

Patent Literature 1: Japanese Patent Application Laid-Open No. 2000-345146
Patent Literature 2: Japanese Patent Application Laid-Open No. 2013-203663

### SUMMARY OF INVENTION

### Technical Problem

The present invention has been made in view of the foregoing circumstances and as a result of intensive studies conducted by the inventors of the present invention on a cracking aerosol composition including carbon dioxide and a liquefied petroleum gas. The present invention has as its object the provision of a cracking aerosol composition which has cracking characteristics in that a foam-breaking sound is generated with the spontaneous breakage of foam formed by foaming generated by the action of carbon dioxide and the liquefied petroleum gas in a flowable discharge and which can reduce the proportion of the liquefied petroleum gas.

### Solution to Problem

The cracking aerosol composition of the present invention is a cracking aerosol composition to be filled into an aerosol container including a pressure-resistant container having an aerosol valve, wherein
the cracking aerosol composition contains a liquefied petroleum gas, water, a defoaming oil, a lower alcohol, carbon dioxide, an emulsifier and an emulsifying aid,
the proportion of the liquefied petroleum gas is 20.0 to 55.0 mass %, the proportion of the defoaming oil is 0.5 to 12.0 mass %, the proportion of the lower alcohol is 5.0 to 30.0 mass %, the proportion of the carbon dioxide is 0.1 to 3.5 mass % and the proportion of the emulsifier is 0.02 to 1.2 mass %, and
the emulsifier includes two types of nonionic surfactants having different HLB values, and the mass ratio of the two types of nonionic surfactants is 1:10 to 10:1.

In the cracking aerosol composition of the present invention, the two types of nonionic surfactants constituting the emulsifier may preferably include a first nonionic surfactant having an HLB value of 15.0 to 18.0 and a second nonionic surfactant having an HLB value of 10.0 to 15.0 and the difference in HLB value between the first nonionic surfactant and the second nonionic surfactant may preferably be 1.0 to 5.0.

In the cracking aerosol composition of the present invention having such a configuration, the first nonionic surfactant may preferably be one or two or more types selected from the group consisting of polyoxyethylene coconut oil fatty acid sorbitan and polyoxyethylene sorbitol monolaurate, and
the second nonionic surfactant may preferably be one or two or more types selected from the group consisting of polyoxyethylene sorbitol tetraoleate and polyoxyethylene hydrogenated castor oil.

In the cracking aerosol composition of the present invention, a liquid concentrate including the water, the lower alcohol, the emulsifier, the defoaming oil and the emulsifying aid may preferably have a viscosity at a temperature of 20°C of 50 to 1000 mPa·s.

In the cracking aerosol composition of the present invention, the defoaming oil may preferably be one or two or more types selected from the group consisting of a silicone oil and a fatty acid ester oil.

In the cracking aerosol composition of the present invention, the defoaming oil may preferably have a compatibility with the liquefied petroleum gas.

### ADVANTAGEOUS EFFECTS OF INVENTION

The cracking aerosol composition of the present invention contains the defoaming oil and carbon dioxide in respective specific proportions and also contains the lower alcohol, the emulsifier and the liquefied petroleum gas in respective specific proportions. Further, as the emulsifier, two types of nonionic surfactants having different HLB values are combined in a specific mass ratio and used, and so the emulsifier sufficiently exerts an emulsifying action even with the defoaming oil and carbon dioxide contained therein, whereby a good emulsified state can be obtained. This makes it possible to obtain a flowable discharge even with a small proportion of the liquefied petroleum gas, and, in such a flowable discharge, the foam-breaking sound is generated with the spontaneous breakage of foam formed by foaming gradually generated by vaporization of the carbon dioxide and the liquefied petroleum gas.

Thus, in the cracking aerosol composition of the present invention, it is possible to obtain the sufficient cracking characteristics in the flowable discharge even with a small proportion of the liquefied petroleum gas. As a result, the amount of the liquid concentrate can be relatively increased by reducing the proportion of the liquefied petroleum gas, and so it is possible to increase the number of usable times of the aerosol product prepared by filling the cracking composition of the present invention into an aerosol container. Further, the proportion of the liquefied petroleum gas having large temperature dependency of pressure (vapor pressure) is reduced by using carbon dioxide that has extremely small temperature dependency of pressure, and so a reduction in temperature dependency in a discharge state is achieved, thereby making it possible to obtain the flowable discharge having stable properties regardless of the use environment. Further, the aerosol product according to the cracking composition of the present invention has a small proportion of the flammable liquefied petroleum gas, and so a reduction in an igniting property and an explosive property is achieved, thereby making it possible to obtain high safety regardless of the use environment.

### DESCRIPTION OF EMBODIMENTS

The cracking aerosol composition of the present invention contains a liquefied petroleum gas, a defoaming oil, a lower alcohol, carbon dioxide and an emulsifier in respective specific proportions and also contains water and an emulsifying aid, and is filled into an aerosol container including a pressure-resistant container having an aerosol valve, thereby producing an aerosol product.

Further, in the cracking aerosol composition of the present invention, not only the liquefied petroleum gas and carbon dioxide constitute a gas phase as a propellant, but also a part of the liquefied petroleum gas and a part of the carbon dioxide constitute a liquid phase together with a liquid concentrate containing water, the defoaming oil, the lower alcohol, the emulsifier and the emulsifying aid. In the discharge, a foam-breaking sound is generated by the action of the liquefied petroleum gas and carbon dioxide. More specifically, the cracking aerosol composition of the present invention forms a flowable discharge, and in the flowable discharge, carbon dioxide and the liquefied petroleum gas are vaporized to cause the discharge to gradually generate foam. Then, the foam formed by the foaming spontaneously breaks to generate a crackling foam-breaking sound. In other words, the flowable discharge has cracking characteristics (i.e., characteristics in which the formed foam breaks with a crackling foam-breaking sound).

The cracking aerosol composition of the present invention is characterized in that the proportion of the liquefied petroleum gas is 20.0 to 55.0 mass %, the proportion of the defoaming oil is 0.5 to 12.0 mass %, the proportion of the lower alcohol is 5.0 to 30.0 mass %, the proportion of the carbon dioxide is 0.1 to 3.5 mass % and the proportion of the emulsifier is 0.02 to 1.2 mass %. The cracking aerosol composition of the present invention is also characterized in that the emulsifier includes two types of nonionic surfactants having different HLB values (Hydrophile-Lipophile Balance: balance between hydrophilic and lipophilic properties) and the mass ratio of the two types of nonionic surfactants is 1:10 to 10:1.

In the cracking aerosol composition of the present invention, the respective proportions of the liquefied petroleum gas, the defoaming oil, the lower alcohol, the carbon dioxide and the emulsifier are appropriately determined within the above-mentioned ranges in accordance with usage or the like in relation to other constituent components.

Specifically, for example, when the proportion of the liquefied petroleum gas is relatively large, the proportion of the emulsifier may preferably be relatively large and the proportion of the lower alcohol may preferably be relatively small. In such a case, the proportion of the emulsifying aid may preferably be large.

When the proportion of the lower alcohol is relatively large, the proportion of the emulsifier may preferably be relatively large.

When the proportion of the emulsifier is relatively large, the proportion of the lower alcohol may preferably be relatively large. When the proportion of the emulsifying aid is small, the proportion of the emulsifier may preferably be relatively large.

When the proportion of the defoaming oil is relatively large, the proportions of the emulsifier and the emulsifying aid may preferably be relatively large.

Hereinafter, components constituting the cracking aerosol composition of the present invention will be described.

In the cracking aerosol composition of the present invention, the liquid phase is composed of an oily phase containing a part of the liquefied petroleum gas dissolved in the lower alcohol and the defoaming oil and an aqueous phase containing water in which a part of carbon dioxide is dissolved. The liquid phase is an oil-in-water type (O/W type) emulsion in which dispersed particles of the oily phase are uniformly dispersed in a dispersion medium of the aqueous phase by the action of the emulsifier and the emulsifying aid.

Water:
Purified water is used as the water which is an essential component.

The proportion of water is appropriately determined in accordance with the proportions of the other components to constitute the cracking aerosol composition in consideration of the use applications of the cracking aerosol composition and the like.

Defoaming oil:
The defoaming oil, which is an essential component, has an action of causing the foam gradually formed in the flowable discharge to spontaneously break.

This defoaming oil is compatible with the liquefied petroleum gas.

The defoaming oil may preferably be one or two or more types selected from the group consisting of a silicone oil and a fatty acid ester oil from the viewpoints of a defoaming property and safety to the human body,.

As specific examples of the silicone oil used as the defoaming oil, may be mentioned dimethicone, cyclopentasiloxane, trisiloxane and caprylylmethicone. Among these, cyclopentasiloxane may be preferable.

As specific examples of the fatty acid ester oil used as the defoaming oil, may be mentioned monovalent alcohol esters such as isopropyl myristate, butyl myristate, isocetyl myristate, octyldodecyl myristate, butyl stearate, ethylhexyl stearate, isopropyl isostearate, isopropyl palmitate, ethylhexyl palmitate, ethyl lynolate, butyloctyl salicylate, cetyl ethylhexanoate and an ethyl olive fatty acid ester (ethyl oleate), and polyhydric alcohol fatty acid esters such as triethylhexanoin, propylene glycol monocaprylate, propylene glycol dicaprylate, trimethylolpropane triethylhexanoate, trimethylolpropane triisostearate and glyceryl tri(caprylate/caprate). Among these, isopropyl myristate, isopropyl palmitate and cetyl ethylhexanoate may be preferable.

In the cracking aerosol composition of the present invention, as the defoaming oil, isopropyl myristate and isopropyl palmitate may be preferable, and isopropyl myristate may particularly be preferable.

As the defoaming oil, ester oils other than the fatty acid ester oils, hydrocarbon oils such as liquid paraffin and vegetable oils such as olive oil can be used.

Herein, as examples of the ester oils other than the fatty acid ester oils, may be mentioned polybasic acid esters such as diisopropyl adipate, diisopropyl sebacate and diethyl sebacate.

The proportion of the defoaming oil is 0.5 to 12.0 mass %, preferably 0.5 to 6.0 mass %, in the entire cracking aerosol composition.

If the proportion of the defoaming oil is too large, there is a possibility that a good emulsified state and emulsifying stability may not be obtained.

On the other hand, if the proportion of the defoaming oil is too small, there is a possibility that the discharge obtained may become foamy and the cracking characteristic may not be obtained.

Lower alcohol:
The lower alcohol, which is an essential component, mainly functions as a solvent for the liquefied petroleum gas and also exhibits a defoaming action and a freezing point lowering action. The lower alcohol having the freezing point lowering action can prevent the discharge from being transformed into a state of sherbet caused by vaporization heat of the liquefied petroleum gas. Further, when the cracking aerosol composition is applied to the human body, in particular, the lower alcohol exhibits a cooling action and a non-residual sensation action in the applied site.

As examples of the lower alcohols, may be mentioned alcohols having 1 to 5 carbon atoms, such as ethanol, 1-propanol and 2-propanol. These may be used either singly or in any combination thereof.

Among these, ethanol may be preferable because solubility and cracking characteristics of the liquefied petroleum gas can be reliably obtained. Further, when the cracking aerosol composition is used as a composition for the human body, use of ethanol as the lower alcohol can provide a favorable feeling of use from the viewpoints of cooling property, non-residual feeling property, and the like.

The proportion of the lower alcohol is 5.0 to 30.0 mass %, preferably 10.0 to 25.0 mass %, in the entire cracking aerosol composition.

If the proportion of the lower alcohol is too large, there is a possibility that an emulsified state may not be obtained. In addition, there is a possibility that foaming may hardly occur in the flowable discharge.

On the other hand, if the proportion of the lower alcohol is too small, there is a possibility that the flowable discharge may not be obtained due to the fact that a sufficient freezing point lowering action by the lower alcohol may not be obtained. More specifically, there is a possibility that the discharge may become a sherbet-like state due to freezing of water in the discharge. In addition, there is a possibility that the liquefied petroleum gas may not be sufficiently dissolved in the liquid concentrate, and due to this, a good emulsified state may not be obtained. In particular, there is a possibility that, if it is applied to the skin surface as a composition for the human body, a favorable feeling of use may not be obtained due to the fact that the feeling of stickiness occurs at the application site.

Emulsifier:
The emulsifier, which is an essential component, has an action of dispersing dispersed particles of the oily phase in a dispersion medium of the aqueous phase.

The emulsifier is composed of two types of nonionic surfactants having different HLB values.

In this emulsifier, the mass ratio of the two types of nonionic surfactants may preferably be 1:10 to 10:1, more preferably 1:5 to 5:1.

In the cracking aerosol composition of the present invention, the two types of nonionic surfactants having different HLB values are used in combination as an emulsifier. Thus, the emulsifying action by the emulsifier is sufficiently exhibited even in the presence of the defoaming oil and carbon dioxide, and so a good emulsified state can be obtained. When the mass ratio of the two types of nonionic surfactants falls within the aforementioned range, good emulsifying stability can be obtained in the composition.

The two types of nonionic surfactants constituting the emulsifier may preferably be composed of a first nonionic surfactant having an HLB value of 15.0 to 18.0 and a second nonionic surfactant having an HLB value of 10.0 to 15.0, and may preferably have a difference of 1.0 to 5.0 between the HLB value of the first nonionic surfactant and the HLB value of the second nonionic surfactant.

When the two types of nonionic surfactants having specific HLB values and a difference in HLB value between them falling within a specific range are used in combination, there can be provided a more excellent emulsifying action. Therefore, it is possible to obtain more excellent emulsifying easiness and emulsifying stability as well as more excellent cracking characteristics. In particular, when the cracking aerosol composition of the present invention is applied to the human body, since the amount of the emulsifier used can be made small, the occurrence of a sticky feeling can be suppressed, and so a favorable feeling of use can be obtained.

Thus, if the difference between the HLB value of the first nonionic surfactant and the HLB value of the second nonionic surfactant is too large or too small, there is a possibility that a good emulsified state and emulsifying stability may not be obtained in either case.

As specific examples of the first nonionic surfactant, may be mentioned polyoxyethylene sorbitan fatty acid esters such as polyoxyethylene (20) sorbitan monolaurate (HLB value: 16.7), polyoxyethylene (20) sorbitan monopalmitate (HLB value: 15.6) and polyoxyethylene (20) coconut oil fatty acid sorbitan (HLB value: 16.9), polyoxyethylene alkyl ethers such as polyoxyethylene (25) octyldodecyl ether (HLB value: 15.7), polyoxyethylene sorbitol fatty acid esters such as polyoxyethylene (6) sorbitol monolaurate (HLB value: 15.5), polyoxyethylene lanolin alcohols such as polyoxyethylene (40) lanolin alcohol (HLB value: 17.0), polyethylene glycol fatty acid esters such as polyethylene glycol distearate (HLB value: 16.5), polyoxyethylene sterols such as polyoxyethylene (20) phytosterol (HLB value: 15.5) and polyoxyethylene alkyl ether phosphoric acids such as polyoxyethylene lauryl ether (10) sodium phosphate (HLB value: 17.0).

As specific examples of the second nonionic surfactant, may be mentioned polyoxyethylene sorbitan fatty acid esters such as polyoxyethylene (85) sorbitan trioleate (HLB value: 11.0) and polyoxyethylene (65) sorbitan tristearate (HLB value: 10.5), polyoxyethylene sorbitol fatty acid esters such as polyoxyethylene (60) sorbitol tetraoleate (HLB value: 14.0), polyoxyethylene alkyl ethers such as polyoxyethylene (10) cetyl ether (HLB value: 13.5) and polyoxyethylene (10) oleyl ether (HLB value: 14.5), polyoxyethylene higher alcohol ether (HLB value: 10.5 to 13.3), polyoxyethylene hydrogenated castor oils such as polyoxyethylene (40) hydrogenated castor oil (HLB value: 12.5), polyglycerin fatty acid esters such as decaglyceryl monomyristate (HLB value: 14.5), polyoxyethylene lanolin such as polyoxyethylene (10) lanolin (HLB value: 12.0), polyoxyethylene alkyl ether phosphoric acid/phosphate such as sodium polyoxyethylene (8) oleyl ether phosphate (HLB value: 12.5) and sodium polyoxyethylene (4) lauryl ether phosphate (HLB value: 13.0), polyethylene glycol fatty acid esters such as polyethylene (10) glycol monolaurate (HLB value: 12.5), polyoxyethylene glycerin fatty acid esters such as polyoxyethylene (15) glyceryl monostearate (HLB value: 13.5) and polyoxyethylene sterols such as polyoxyethylene (10) phytosterol (HLB value: 12.5).

As a preferable combination of the first nonionic surfactant and the second nonionic surfactant, it is preferable that the first nonionic surfactant is one or two or more types selected from the group consisting of polyoxyethylene coconut oil fatty acid sorbitan and polyoxyethylene sorbitol monolaurate, and the second nonionic surfactant is one or two or more types selected from the group consisting of polyoxyethylene sorbitol tetraoleate and polyoxyethylene hydrogenated castor oil.

Specifically, it is preferable to use polyoxyethylene (20) coconut oil fatty acid sorbitan as the first nonionic surfactant and polyoxyethylene (60) sorbitol tetraoleate as the second nonionic surfactant, or use polyoxyethylene (6) sorbitol monolaurate as the first nonionic surfactant, and polyoxyethylene (40) hydrogenated castor oil as the second nonionic surfactant.

In the first nonionic surfactant and the second nonionic surfactant, as described above, the mass ratio, specifically, the ratio of the proportion of the first nonionic surfactant in the cracking aerosol composition and the proportion of the second nonionic surfactant in the cracking aerosol composition (the first nonionic surfactant : the second nonionic surfactant) may preferably be 1:10 to 10:1, more preferably 1:5 to 5:1, particularly preferably 1:2 to 2:1.

The proportion of the emulsifier is 0.02 to 1.2 mass %, preferably 0.20 to 0.80 mass %, in the entire cracking aerosol composition.

If the proportion of the emulsifier is too large, there is a possibility that the discharge may become foamy and the cracking characteristics may not be obtained. In particular, if it is applied to the skin surface as a composition for the human body, there is a possibility that a favorable feeling of use may not be obtained due to a feeling of stickiness or a strong skin irritation at the application site.

On the other hand, if the proportion of the emulsifier is too small, there is a possibility that an emulsified state may not be obtained.

Emulsifying aid:
The emulsifying aid, which is an essential component, is a powder, exists in a dispersed state in the liquid phase, and has an action of facilitating dispersion of the dispersed particles of the oily phase in a dispersion medium of the aqueous phase, and an action of improving the stability of the dispersed state, that is, the emulsifying stability.

As examples of the material of the powder constituting the emulsifying aid, may be mentioned silica, talc, alumina, zeolite and polyamide (nylon).

As the material of the powder constituting the emulsifying aid in the cracking aerosol composition of the present invention, silica may be preferable.

The average particle size of the powder constituting the emulsifying aid may preferably be 1 to 30 µm.

If the average particle size of the powder constituting the emulsifying aid is too large, there is a possibility that a feeling of roughness may occur at the application site particularly when the cracking aerosol composition is applied to the skin surface as a composition for the human body.

On the other hand, if the average particle size of the powder constituting the emulsifying aid is too small, there is a possibility that the cracking aerosol composition may adversely affect the human body particularly when it is applied to the skin surface as a composition for human body.

The proportion of the emulsifying aid may preferably be 0.4 to 0.8 mass % in the entire cracking aerosol composition.

If the proportion of the emulsifying aid is too large, clogging of the aerosol valve tends to occur, and there is a possibility that a favorable feeling of use may not be obtained particularly when the cracking aerosol composition is applied to the skin surface as a composition for the human body, because a feeling of roughness may occur at the application site.

On the other hand, if the proportion of the emulsifying aid is too small, there is a possibility that the emulsifying easiness may not be obtained. In particular, long shaking periods may be required to obtain a good emulsified state in the cracking aerosol composition.

Liquefied petroleum gas:
The liquefied petroleum gas, which is an essential component, is partly dissolved in the liquid concentrate to constitute the liquid phase, and also forms the gas phase as a propellant together with carbon dioxide (specifically, a part of carbon dioxide) by another part thereof.

As specific examples of the liquefied petroleum gases, may be mentioned propane, n-butane, iso-butane and mixtures thereof. The liquefied petroleum gas may contain n-pentane and iso-pentane.

The proportion of the liquefied petroleum gas is 20.0 to 55.0 mass %, preferably 25.0 to 55.0 mass %, in the entire cracking aerosol composition.

If the proportion of the liquefied petroleum gas is too small, there is a possibility that sufficient cracking characteristics may not be obtained because the discharge may become foamy, or, although the formed foam in the discharge may spontaneously break with a fizzy sound, a large crackling sound is not obtained.

Carbon dioxide:
Since carbon dioxide, which is an essential component, has solubility in water, a part thereof is dissolved in the liquid concentrate to constitute the liquid phase, and also forms the gas phase as a propellant together with the liquefied petroleum gas (specifically, a part of the liquefied petroleum gas) by another part thereof.

The proportion of carbon dioxide is 0.1 to 3.5 mass %, preferably 0.8 to 2.2 mass %, in the entire cracking aerosol composition.

If the proportion of carbon dioxide is too large, there is a possibility that sufficient safety may not be obtained due to too large internal pressure of the product in the aerosol container.

On the other hand, if the proportion of carbon dioxide is too small, there is a possibility that sufficient cracking characteristics may not be obtained. In addition, there is a possibility that a good discharge state may not be obtained particularly in a low temperature environment.

Here, in the cracking aerosol composition of the present invention, the internal pressure of the product in the aerosol container may preferably be not more than 0.8 MPa, more preferably 0.3 to 0.7 MPa, at a temperature of 35°C.

When the internal pressure of the product is too high, there is a possibility that sufficient safety may not be obtained due to too high filling pressure of the propellant (specifically, liquefied petroleum gas and carbon dioxide).

Thus, the product internal pressure of 0.3 to 0.7 MPa at a temperature of 35°C in the aerosol container can provide sufficient safety required for the aerosol product as well as sufficient cracking characteristics.

Optional component:
The cracking aerosol composition of the present invention may contain an optional component as needed in addition to the essential components (specifically, water, the defoaming oil, the lower alcohol, the emulsifier, the emulsifying aid, the liquefied petroleum gas and carbon dioxide). As specific examples thereof, may be mentioned a thickener, a moisturizer, a fungicide and a preservative, a skin protectant (amino acid), vitamins, various extracts, a deodorant, a cooling agent, an ultraviolet absorber, an ultraviolet light scattering agent, a pest-repelling ingredient, perfume and others.

As the thickener constituting the optional component, a cellulose-based polymer and a water-soluble polymer such as a gum substance are used.

As examples of the cellulose-based polymer constituting the thickener, may be mentioned hydroxyethyl cellulose, hydroxypropyl cellulose, hydroxypropyl methylcellulose, sodium carboxymethyl cellulose, nitrocellulose and crystalline cellulose.

As examples of the gum substance constituting the thickener, may be mentioned xanthan gum, carrageenan, gum arabic, gum traganth, cationic guar gum, guar gum, gellan gum and locust bean gum.

As the thickener in the cracking aerosol composition of the present invention, the water-soluble polymers may be used either singly or in any combination thereof.

The proportion of the thickener may preferably be 0.04 to 1.0 mass % in the entire cracking aerosol composition.

If the proportion of the thickener is too large, the viscosity of the cracking aerosol composition (liquid concentrate) becomes too high, which makes it difficult to sufficiently disperse the dispersed particles of the oily phase in the dispersion medium of the aqueous phase. As a result, there is a possibility that a good emulsified state may not be obtained in the cracking aerosol composition. Further, there is a possibility that a good discharge state may not be obtained. Specifically, there is a possibility that discharging itself may not be performed or an expected amount of the discharge may not be obtained. Further, if the cracking aerosol composition of the present invention is applied to the human body, in particular, there is a possibility that a favorable feeling of use may not be obtained due a feeling of stickiness occurring.

On the other hand, if the proportion of the thickener is too low, the viscosity of the cracking aerosol composition (liquid concentrate) becomes too small, which makes it difficult to obtain a good emulsified state in the cracking aerosol composition. As a result, there is a possibility that the cracking characteristics in the discharge may not be obtained.

Further, as examples of the moisturizer constituting the optional component, may be mentioned propylene glycol, glycerin, 1,3-butylene glycol, collagen, xylitol, sorbitol, hyaluronic acid, sodium lactate, keratin, casein, lecithin and urea.

As examples of the fungicides and preservatives constituting the optional component, may be mentioned paraoxybenzoic acid esters, sodium benzoate, phenoxyethanol, benzalkonium chloride, benzethonium chloride, chlorhexidine and chlorhexidine chloride.

As examples of the skin protectants (amino acid) constituting the optional component, may be mentioned glycine, alanine, isoleucine, leucine, serine, tryptophan, cystine, methionine, aspartic acid, glutamic acid and arginine.

As examples of the vitamins constituting the optional component, may be mentioned retinol, retinyl palmitate, pyridoxine hydrochloride, tocopherol nicotinate, tocopherol acetate, vitamin D₂, pantothenic acid and biotin.

As examples of the various extracts constituting the optional component, may be mentioned peony extract, luffa cylindrica extract, rose extract, lemon extract, aloe extract, calamus root extract, eucalyptus globules extract, sage extract, tea extract, seaweed extract, placenta extract and silk extract.

As examples of the deodorants constituting the optional component, may be mentioned lauryl methacrylate, geranyl crotolate, acetophenone myristate, benzyl acetate, benzyl propionate, methyl benzoate and methyl phenylacetate.

As examples of the cooling agent constituting the optional component, may be mentioned l-menthol and camphor.

In the cracking aerosol composition constituted by the above-mentioned essential components and optional components, the viscosity of the liquid concentrate at a temperature of 20°C may preferably be 50 to 1000 mPa·s, more preferably 80 to 300 mPa·s.

If the viscosity of the liquid concentrate is too high, there is a possibility that a good discharge state may not be obtained. Specifically, there is a possibility that the discharge itself may not be performed, or that the discharge in a desired amount may not be obtained. In addition, particularly in the case of application to the human body, there is a possibility that a feeling of stickiness may occur and a favorable feeling of use may not be obtained.

On the other hand, if the viscosity of the liquid concentrate is too small, there is a possibility that a good emulsified state may not be obtained, and sufficient cracking characteristics may not be obtained.

The cracking aerosol composition of the present invention with such a configuration can be produced by filling an aerosol container with the essential components (specifically, water, the defoaming oil, the lower alcohol, the emulsifier, the emulsifying aid, the liquefied petroleum gas and carbon dioxide) and the optional component contained as necessary, followed by shaking.

In such a cracking aerosol composition of the present invention, the defoaming oil and carbon dioxide are contained in respective specific proportions, and the lower alcohol, the emulsifier and the liquefied petroleum gas are also contained in respective specific proportions. Further, two types of nonionic surfactants having different HLB values are combined in a specific mass ratio and used as the emulsifier, and so the emulsifier sufficiently exerts an emulsifying action even in the presence of the defoaming oil and carbon dioxide. As a result, a good emulsified state can be obtained. This makes it possible to obtain a flowable discharge even with a small proportion of the liquefied petroleum gas. In such a flowable discharge, a foam-breaking sound (crackling sound) is generated with the spontaneous breakage of foam formed by foaming gradually generated by vaporization of the carbon dioxide and the liquefied petroleum gas.

Thus, in the cracking aerosol composition of the present invention, it is possible to obtain the sufficient cracking characteristics in the flowable discharge even with a small proportion of the liquefied petroleum gas. Specifically, the foam formed in the flowable discharge spontaneously breaks with a crackling sound.

As a result, in the cracking aerosol composition of the present invention, the amount of the liquid concentrate can be relatively increased by reducing the proportion of the liquefied petroleum gas, and so it is possible to increase the number of usable times of the aerosol product prepared by filling the cracking composition into an aerosol container. Further, the proportion of the liquefied petroleum gas having large temperature dependency of pressure (vapor pressure) is reduced by using carbon dioxide that has extremely small temperature dependency of pressure, and so a reduction in temperature dependency in a discharge state is achieved, thereby making it possible to obtain the flowable discharge having stable properties regardless of the use environment. Specifically, the good discharge state can be obtained under a low-temperature environment. Further, the aerosol product according to the cracking composition of the present invention has a small proportion of the flammable liquefied petroleum gas, and so a reduction in an igniting property and an explosive property is achieved, thereby making it possible to obtain high safety regardless of the use environment.

Further, when the cracking aerosol composition of the present invention is applied to the human body, in particular, the cracking characteristics can cause a refreshing feeling, and so a good use feeling can be obtained.

Note that the results of experiments conducted by the inventors have revealed that the proportion of the liquefied petroleum gas can be reduced in the cracking aerosol composition including the liquefied petroleum gas and carbon dioxide by formulating the defoaming oil, using two types of nonionic surfactants having different HLB values in a specific mass ratio as the emulsifier and using the defoaming oil, the lower alcohol, the emulsifier, the liquefied petroleum gas and carbon dioxide in respective specific proportions.

Further, the cracking aerosol composition of the present invention can be used in various applications, for example, for the human body or the like. The composition can be particularly suitably used as a composition for the human body as a moisturizing action can be obtained due to the defoaming oil and a blood circulation-promoting effect or the like can be expected due to carbon dioxide.

Specifically, the composition can be used as a body lotion agent, a skin care agent, a hair tonic, a massage agent, a hair styling agent, a hair treatment agent, a shampoo agent, a conditioner agent, a repellent, an ultraviolet absorber, an ultraviolet light scattering agent and the like.

### Examples

Hereinafter, Examples of the present invention will be described, however, the present invention is not limited by these Examples.

Examples 1 to 16 and Comparative Examples 1 to 4:
First, composition materials shown in Table 1 and Table 2 were prepared and a liquid concentrate was prepared by mixing the composition materials except the liquefied petroleum gas and carbon dioxide in respective proportions indicated in the Table 1 and Table 2. The viscosity at 20°C of the liquid concentrate thus obtained was measured. The results are shown in Table 1 and Table 2.

Next, the obtained liquid concentrate, the liquefied petroleum gas (0.15 MPa) and carbon dioxide were filled into an aerosol container, which is a transparent pressure-resistant container made of glass and having an aerosol valve, in respective proportions indicated in Table 1 and Table 2 so that the mass of the content became 40 g, thereby producing an aerosol product for evaluation.

A plurality of the aerosol products for evaluation thus obtained each had a product internal pressure at a temperature of 35°C within a range of 0.3 to 0.7 MPa.

In Example 1 to Example 13 and Comparative Example 1 to Comparative Example 4, the mass ratio of two types of nonionic surfactants (nonionic surfactant (1) :nonionic surfactant (2)) was 1.9:1 and the difference in HLB value between the nonionic surfactant (1) as a first nonionic surfactant and the nonionic surfactant (2) as a second nonionic surfactant was 2.9.

Further, in Example 14, the mass ratio of two types of nonionic surfactants (nonionic surfactant (3) :nonionic surfactant (4)) was 1.9:1 and the difference in HLB value between the nonionic surfactant (3) as a first nonionic surfactant and the nonionic surfactant (4) as a second nonionic surfactant was 3.

Further, in Example 15, the mass ratio of two types of nonionic surfactants (nonionic surfactant (1) :nonionic surfactant (2)) was 1:1.1, and in Example 16, the mass ratio of two types of nonionic surfactants (nonionic surfactant (1) :nonionic surfactant (2)) was 1:1.9.

The obtained aerosol products for evaluation were each subjected to the following evaluation. The results are shown in Table 1 and Table 2.

Emulsion formability: easiness of emulsification:
The obtained aerosol products for evaluation were shaken under a temperature condition of room temperature (25°C) (hereinafter, also referred to as "room temperature condition") and a shaking condition of 60 times/min, and emulsion formability (easiness of emulsification) was evaluated by confirming the number of times of shaking necessary for forming an emulsion.

Emulsion reformability: emulsion stability:
The obtained aerosol products for evaluation were shaken under the room temperature condition to form an emulsion and then left to stand under the room temperature condition for one day. After that, the products were shaken under the room temperature condition and the shaking condition of 60 times/min, and emulsion reformability (emulsion stability) was evaluated by confirming the number of times of shaking necessary for forming an emulsion.

Foam-breaking property: cracking characteristics:
The obtained aerosol products for evaluation were shaken for 3 minutes under the room temperature condition and the shaking condition of 60 times/min and then left to stand for one hour. Subsequently, the aerosol products for evaluation were shaken for 10 seconds under the room temperature condition and the shaking condition of 60 times/min, and, then, 1 g of the content was discharged to confirm the property of the formed discharge. The foam-breaking property (cracking characteristics) was evaluated on the basis of the following evaluation criteria: the composition is evaluated as "A" for having extremely excellent cracking characteristics of the flowable discharge when foaming is gradually generated in the flowable discharge and all of the foam formed by the foaming spontaneously breaks with a crackling sound; the composition is evaluated as "B" for having good cracking characteristics of the flowable discharge when most of the foam formed by the foaming that is gradually generated in the flowable discharge spontaneously breaks with a crackling sound; the composition is evaluated as "C" for having cracking characteristics of the flowable discharge when some of the foam formed by the foaming that is gradually generated in the flowable discharge spontaneously breaks with a crackling sound; and the composition is evaluated as "D" for having poor cracking characteristics of the discharge when there is no crackling sound in the discharge.

**Table 1:**

| | | | Example 1 | Example 2 | Example 3 | Example 4 | Example 5 | Example 6 | Example 7 | Example 8 | Example 9 | Example 10 | Example 11 | Example 12 | Example 13 | Example 14 | Example 15 | Example 16 |
|---|---|---|---|---|---|---|---|---|---|---|---|---|---|---|---|---|---|---|
| | Defoaming oil (Mass %) | Isopropyl myristate | 2.65 | 3.65 | 0.58 | 11.60 | 2.90 | 2.90 | 2.90 | 2.90 | 2.90 | 2.90 | - | - | - | 0.58 | 0.58 | 0.58 |
| | | Isopropyl palmitate | - | - | - | - | - | - | - | - | - | - | 2.90 | - | - | - | - | - |
| | | Cetyl isooctanoate | - | - | - | - | - | - | - | - | - | - | - | - | 2.90 | - | - | - |
| | | Cyclopentasiloxane | - | - | - | - | - | - | - | - | - | - | - | 2.90 | - | - | - | |
| | Emulsifier (Mass %) | Nonionic surfactant(1) (HLB value 16.9) | 0.21 | 0.29 | 0.23 | 0.23 | 0.23 | 0.23 | 0.23 | 0.23 | 0.23 | 0.23 | 0.23 | 0.23 | 0.23 | - | 0.17 | 0.12 |
| | | Nonionic surfactant(2) (HLB value 14.0) | 0.11 | 0.15 | 0.12 | 0.12 | 0.12 | 0.12 | 0.12 | 0.12 | 0.12 | 0.12 | 0.12 | 0.12 | 0.12 | - | 0.18 | 0.23 |
| | | Nonionic surfactant(3) (HLB value 15.5) | - | - | - | - | - | - | - | - | - | - | - | - | - | 0.23 | - | - |
| Composition | | Nonionic surfactant(4) (HLB value 12.5) | - | - | - | - | - | - | - | - | - | - | - | - | - | 0.12 | - | - |
| | | Total of emulsifiers | 0.32 | 0.44 | 0.35 | 0.35 | 0.35 | 0.35 | 0.35 | 0.35 | 0.35 | 0.35 | 0.35 | 0.35 | 0.35 | 0.35 | 0.35 | 0.35 |
| | Emulsifying aid (Mass %) | Silica powder (Average particle size 30µm) | 0.48 | 0.66 | 0.52 | 0.52 | 0.52 | 0.52 | 0.52 | 0.52 | 0.52 | 0.52 | 0.52 | 0.52 | 0.52 | 0.52 | 0.52 | 0.52 |
| | Lower alcohol (Mass %) | Ethanol | 10.60 | 14.60 | 11.60 | 11.60 | 23.20 | 8.70 | 11.60 | 11.60 | 11.60 | 11.60 | 11.60 | 11.60 | 11.60 | 11.60 | 11.60 | 11.60 |
| | Thickener (Mass %) | Hydroxyethyl cellulose | 0.27 | 0.37 | 0.29 | 0.29 | 0.29 | 0.29 | 0.29 | 0.29 | 0.29 | 0.29 | 0.29 | 0.29 | 0.29 | 0.29 | 0.29 | 0.29 |
| | Moisturizer (Mass %) | 1,3-Butylene glycol | 2.65 | 3.65 | 2.90 | 2.90 | 2.90 | 2.90 | 2.90 | 2.90 | 2.90 | 2.90 | 2.90 | 2.90 | 2.90 | 2.90 | 2.90 | 2.90 |
| | Water (Mass %) | | 36.03 | 49.63 | 41.76 | 30.74 | 27.84 | 42.34 | 59.44 | 41.34 | 37.94 | 39.44 | 39.44 | 39.44 | 39.44 | 41.76 | 41.76 | 41.76 |
| | Liquefied petroleum gas (Mass %) | | 45.00 | 25.00 | 40.00 | 40.00 | 40.00 | 40.00 | 20.00 | 40.00 | 40.00 | 40.00 | 40.00 | 40.00 | 40.00 | 40.00 | 40.00 | 40.00 |
| | Carbon dioxide (Mass %) | | 2.00 | 2.00 | 2.00 | 2.00 | 2.00 | 2.00 | 2.00 | 0.10 | 3.50 | 2.00 | 2.00 | 2.00 | 2.00 | 2.00 | 2.00 | 2.00 |
| | Total (Mass %) | | 100.00 | 100.00 | 100.00 | 100.00 | 100.00 | 100.00 | 100.00 | 100.00 | 100.00 | 100.00 | 100.00 | 100.00 | 100.00 | 100.00 | 100.00 | 100.00 |
| | Viscosity of Liquid Concentrate at 20°C (mPa·s) | | 130 | 130 | 130 | 130 | 130 | 130 | 100 | 120 | 130 | 130 | 130 | 130 | 130 | 130 | 130 | 130 |
| Evaluation | Emulsion Formability | | 5 | 4 | 5 | 6 | 4 | 5 | 3 | 5 | 5 | 5 | 5 | 6 | 6 | 5 | 6 | 6 |
| | Emulsion Reformability | | 3 | 2 | 3 | 4 | 3 | 4 | 2 | 3 | 3 | 3 | 3 | 4 | 4 | 3 | 3 | 4 |
| | Foam Breaking Property | | A | B | A | A | A | B | C | A | A | A | A | A | A | A | A | A |

**Table 2:**

| | | | Comparative example 1 | Comparative example 2 | Comparative example 3 | Comparative example 4 |
|---|---|---|---|---|---|---|
| Composition | Defoaming oil (Mass %) | Isopropyl myristate | - | 2.90 | 2.90 | 2.90 |
| | (Mass %) | Nonionic surfactant (1) (HLB value 16.9) | 0.23 | 0.23 | 0.23 | 0.23 |
| | | Nonionic surfactant(2) (HLB value 14.0) | 0.12 | 0.12 | 0.12 | 0.12 |
| | | Total of emulsifiers | 0.35 | 0.35 | 0.35 | 0.35 |
| | Emulsifying aid (Mass %) | Silica powder (Average particle size 30µm) | 0.52 | 0.52 | 0.52 | 0.52 |
| | Lower alcohol (Mass %) | Ethanol | 11.60 | 2.90 | 34.80 | 11.60 |
| | Thickener (Mass %) | Hydroxyethyl cellulose | 0.29 | 0.29 | 0.29 | 0.29 |
| | Moisturizer (Mass %) | 1,3-Butylene glycol | 2.90 | 2.90 | 2.90 | 2.90 |
| | hater (Mass %) | | 42.34 | 48.14 | 16.24 | 64.44 |
| | Liquefied petroleum gas (Mass %) | | 40.00 | 40.00 | 40.00 | 1500 |
| | ..arbon dioxide (Mass %) | | 2.00 | 2.00 | 2.00 | 2.00 |
| | Total (Mass %) | | 100.00 | 100.00 | 100.00 | 100.00 |
| | Viscosity of Liquid Concentrate at 20°C (mPa▪s) | | 130 | 130 | 130 | 100 |
| Evaluation | Emulsion Formability | | 5 | 8 | - | 3 |
| | Emulsion Reformability | | 3 | 5 | - | 2 |
| | Foam Breaking Property | | D | D | - | D |

In Table 1 and Table 2, the nonionic surfactant (1) is polyoxyethylene (20) coconut oil fatty acid sorbitan (HLB value of 16.9), the nonionic surfactant (2) is polyoxyethylene (60) sorbitol tetraoleate (HLB value of 14.0), the nonionic surfactant (3) is polyoxyethylene (6) sorbitol monolaurate (HLB value of 15.5) and the nonionic surfactant (4) is polyoxyethylene (40) hydrogenated castor oil (HLB value of 12.5).

Further, in Table 2, the aerosol composition according to Comparative Example 3 could not form emulsion, and the emulsion state could not be obtained, and so the emulsion formability, the emulsion reformability and the foam-breaking property could not be evaluated.

From the aforementioned results, it was confirmed that, in the aerosol compositions of Examples 1 to Examples 16 according to the present invention, where carbon dioxide and the liquefied petroleum gas were contained, the easiness of emulsification and the emulsion stability can be favorably obtained and a foam-breaking sound can be generated in the flowable discharge even with a small proportion of the liquefied petroleum gas.

## Claims

1. A cracking aerosol composition to be filled into an aerosol container including a pressure-resistant container having an aerosol valve, wherein
the cracking aerosol composition comprises a liquefied petroleum gas, water, a defoaming oil, a lower alcohol, carbon dioxide, an emulsifier and an emulsifying aid,
a proportion of the liquefied petroleum gas is 20.0 to 55.0 mass %, a proportion of the defoaming oil is 0.5 to 12.0 mass %, a proportion of the lower alcohol is 5.0 to 30.0 mass %, a proportion of the carbon dioxide is 0.1 to 3.5 mass % and a proportion of the emulsifier is 0.02 to 1.2 mass %, and
the emulsifier includes two types of nonionic surfactants having different HLB values, and a mass ratio of the two types of nonionic surfactants is 1:10 to 10:1.

2. The cracking aerosol composition according to claim 1, wherein the two types of nonionic surfactants constituting the emulsifier include a first nonionic surfactant having an HLB value of 15.0 to 18.0 and a second nonionic surfactant having an HLB value of 10.0 to 15.0 and a difference in HLB value between the first nonionic surfactant and the second nonionic surfactant is 1.0 to 5.0.

3. The cracking aerosol composition according to claim 2, wherein
the first nonionic surfactant is one or two or more types selected from the group consisting of polyoxyethylene coconut oil fatty acid sorbitan and polyoxyethylene sorbitol monolaurate, and
the second nonionic surfactant is one or two or more types selected from the group consisting of polyoxyethylene sorbitol tetraoleate and polyoxyethylene hydrogenated castor oil.

4. The cracking aerosol composition according to any one of claims 1 to 3, wherein a liquid concentrate including the water, the lower alcohol, the emulsifier, the defoaming oil and the emulsifying aid has a viscosity at a temperature of 20°C of 50 to 1000 mPa.s.

5. The cracking aerosol composition according to any one of claims 1 to 4, wherein the defoaming oil is one or two or more types selected from the group consisting of a silicone oil and a fatty acid ester oil.

6. The cracking aerosol composition according to any one of claims 1 to 5, wherein the defoaming oil has a compatibility with the liquefied petroleum gas.

## Patentansprüche

1. Knisternde (Cracking)-Aerosol-Zusammensetzung zum Einfüllen in einen Aerosolbehälter, der einen druckfesten Behälter mit einem Aerosolventil umfasst, wobei
die knisternde (Cracking)-Aerosol-Zusammensetzung ein verflüssigtes Erdgas, Wasser, ein Entschäumungsöl, einen niederen Alkohol, Kohlendioxid, einen Emulgator und ein Emulgierhilfsmittel umfasst,
der Anteil des verflüssigten Erdgases 20,0 bis 55,0 Massen-% beträgt, der Anteil des Entschäumungsöls 0,5 bis 12,0 Massen-% beträgt, der Anteil des niederen Alkohols 5,0 bis 30,0 Massen-% beträgt, der Anteil des Kohlendioxids 0,1 bis 3,5 Massen-% beträgt und der Anteil des Emulgators 0,02 bis 1,2 Massen-% beträgt und
der Emulgator zwei Arten von nichtionischen Tensiden mit unterschiedlichen HLB-Werten umfasst und das Massenverhältnis der zwei Arten von nichtionischen Tensiden 1:10 bis 10:1 beträgt.

2. Knisternde (Cracking)-Aerosol-Zusammensetzung gemäß Anspruch 1, wobei die zwei Arten von nichtionischen Tensiden, die den Emulgator bilden, ein erstes nichtionisches Tensid mit einem HLB-Wert von 15,0 bis 18,0 und ein zweites nichtionisches Tensid mit einem HLB-Wert von 10,0 bis 15,0 umfasst und die Differenz der HLB-Werte zwischen dem ersten nichtionischen Tensid und dem zweiten nichtionischen Tensid 1,0 bis 5,0 betrug.

3. Knisternde (Cracking)-Aerosol-Zusammensetzung gemäß Anspruch 2, wobei es sich bei dem ersten nichtionischen Tensid um eine oder zwei oder mehr Arten handelt, die aus der Gruppe ausgewählt sind, die aus Polyoxyethylenkokosnussölfettsäuresorbitan und Polyoxyethylensorbitmonolaurat besteht, und
es sich bei dem zweiten nichtionischen Tensid um eine oder zwei oder mehr Arten handelt, die aus der Gruppe ausgewählt sind, die aus Polyoxyethylensorbittetraoleat und Polyoxyethylen-hydrogeniertem Ricinusöl besteht.

4. Knisternde (Cracking)-Aerosol-Zusammensetzung gemäß einem der Ansprüche 1 bis 3, wobei ein flüssiges Konzentrat, das das Wasser, den niederen Alkohol, den Emulgator, das Entschäumungsöl und das Emulgierhilfsmittel umfasst, eine Viskosität bei einer Temperatur von 20°C von 50 bis 1000 mPa·s aufweist.

5. Knisternde (Cracking)-Aerosol-Zusammensetzung gemäß einem der Ansprüche 1 bis 4, wobei es sich bei dem Entschäumungsöl um eine oder zwei oder mehr Arten handelt, die aus der Gruppe ausgewählt sind, die aus einem Silikonöl und einem Fettsäureesteröl besteht.

6. Knisternde (Cracking)-Aerosol-Zusammensetzung gemäß einem der Ansprüche 1 bis 5, wobei das Entschäumungsöl eine Verträglichkeit mit dem verflüssigten Erdgas aufweist.

## Revendications

1. Composition aérosol de craquage pour remplir un contenant d'aérosol incluant un contenant résistant à la pression ayant une valve d'aérosol, dans laquelle
la composition aérosol de craquage comprend un gaz de pétrole liquéfié, de l'eau, une huile démoussante, un alcool inférieur, du dioxyde de carbone, un émulsifiant et une aide émulsifiante,
une proportion du gaz de pétrole liquéfié est de 20,0 à 55,0 % en masse, une proportion de l'huile démoussante est de 0,5 à 12,0 % en masse, une proportion de l'alcool inférieur est de 5,0 à 30,0 % en masse, une proportion du dioxyde de carbone est de 0,1 à 3,5 % en masse et une proportion de l'émulsifiant est de 0,02 à 1,2 % en masse et
l'émulsifiant inclut deux types de tensioactifs non ioniques ayant des valeurs HLB différentes, et un rapport de masse des deux types de tensioactifs non ioniques est de 1:10 à 10:1.

2. Composition aérosol de craquage selon la revendication 1, dans laquelle les deux types de tensioactifs non ioniques constituant l'émulsifiant incluent un premier tensioactif non ionique ayant une valeur HLB de 15,0 à 18,0 et un second tensioactif non ionique ayant une valeur HLB de 10,0 à 15,0 et une différence de valeur HLB entre le premier tensioactif non ionique et le second tensioactif non ionique est de 1,0 à 5, 0 .

3. Composition aérosol de craquage selon la revendication 2, dans laquelle
le premier tensioactif non ionique est un ou plusieurs types choisis dans le groupe consistant en du sorbitan d'acide gras d'huile de coco polyoxyéthyléné et du monolaurate de sorbitol polyoxyéthyléné, et
le second tensioactif non ionique est un ou deux ou plusieurs types choisis dans le groupe consistant en du tétraoléate de sorbitol polyoxyéthyléné et de l'huile de ricin hydrogénée polyoxyéthylénée.

4. Composition aérosol de craquage selon l'une quelconque des revendications 1 à 3, dans laquelle un concentré liquide incluant l'eau, l'alcool inférieur, l'émulsifiant, l'huile démoussante et l'aide émulsifiante a une viscosité à une température de 20 °C de 50 à 1 000 mPa · s.

5. Composition aérosol de craquage selon l'une quelconque des revendications 1 à 4, dans laquelle l'huile démoussante est un ou deux ou ou plusieurs types choisis dans le groupe consistant en une huile de silicone et une huile d'ester d'acide gras.

6. Composition aérosol de craquage selon l'une quelconque des revendications 1 à 5, dans laquelle l'huile démoussante est compatible avec le gaz de pétrole liquéfié.
